# EUROPEAN PATENT SPECIFICATION

(11) **EP 0 760 602 B1**
(45) Date of publication and mention of the grant of the patent: **13.12.2006**
(21) Application number: 95921459.4
(22) Date of filing: 26.05.1995
(51) Int. Cl.: C07K 14/705, A01N 37/18, A01N 63/00, A61K 38/00, A61K 48/00, C07H 21/04, C07K 1/00, C07K 16/00, C12N 15/00, C07K 14/52, C07K 16/28

(54) **USE OF FAS LIGAND TO SUPPRESS LYMPHOCYTE-MEDIATED IMMUNE RESPONSES**
VERWENDUNG VON FAS-LIGANDEN ZUR UNTERDRÜKUNG LYMPHOZYT-VERMITTELTER IMMUNREAKTIONEN
UTILISATION DU LIGAND FAS COMME DEPRESSEUR DES REPONSES IMMUNITAIRES A MEDIATION LYMPHOCYTAIRE

(30) Priority: 27.05.1994 US 250478; 26.01.1995 US 378507
(43) Date of publication of application: 12.03.1997
(73) Proprietor: Donald Bellgrau, Denver, Colorado 80202 (US); Duke Richard C., Denver, Colorado 80209 (US)
(72) Inventor: BELLGRAU, Donald, Denver, CO 80220 (US); DUKE, Richard, C., Denver, CO 80209 (US)
(74) Representative: Roques, Sarah Elizabeth
(86) International application number: PCT/US1995/006742
(87) International publication number: WO 1995/032627

(56) References cited:
- EP-A- 0 675 200
- WO-A-95/18819
- US-A- 5 219 740
- LYNCH, D.H. ET AL.: "The mouse Fas-ligand gene is mutated in gld mice and is part of a TNF family gene cluster" IMMUNITY, vol. 1, 1994, pages 131-136, XP002054754
- NAGATA, S.: "Fas and Fas Ligand: A death factor and its receptor" ADV. IMMUNOL., vol. 57, 1994, pages 129-144, XP002097379
- MOUNTZ, J.D.: "Autoimmune disease" ARTHRITIS & RHEUMATISM, vol. 37, no. 10, October 1994, pages 1415-1420, XP002097380
- LYNCH, D.H. ET AL.: "Fas and FasL in the homeostatic regulation of immune responses" IMMUNOLOGY TODAY, vol. 16, no. 12, December 1995, pages 569-574, XP002097381
- A. JOHNSTONE et al., "Immunochemistry in Practice", Published 1987, by BLACKWELL SCIENTIFIC PUBLICATIONS (OXFORD), pages 30-47.
- INTERNATIONAL IMMUNOLOGY, Volume 6, No. 10, issued October 1994, TAKAHASHI et al., "Human Fas Ligand: Gene Structure, Chromosomal Location and Species Specificity", pages 1567-1574.
- SCIENCE, Volume 267, issued 10 March 1995, NAGATA et al., "The Fas Death Factor", pages 1449-1456.
- CELL, Volume 76, issued 25 March 1994, TAKAHASHI et al., "Generalized Lymphoproliferative Disease in Mice, Caused by a Point Mutation in the Fas Ligand", pages 969-976.
- FASEB JOURNAL, Volume 8, No. 5, issued 18 March 1994, LEE et al., "Fas Ligand Expression on an Activated Cytotoxic T-Cell Line", page A770, Abstract 4467.
- CELL, Volume 75, issued 17 December 1993, SUDA et al., "Molecular Cloning and Expression of the Fas Ligand, a Novel Member of the Tumor Necrosis Factor Family", pages 1169-1178.
- JOURNAL OF EXPERIMENTAL MEDICINE, Volume 179, issued March 1994, SUDA et al., "Purification and Characterization of the Fas-ligand that Induces Apoptosis", pages 873-879.

## Description

### Field of the Invention

This invention relates to suppression of lymphocyte-mediated immune responses, including those directed against autologous tissue in autoimmune conditions and/or transplanted tissues.

### Background of the Invention

Type I or juvenile onset diabetes is a major health problem in the United States. Diabetes and its complications double the risk of fatal heart disease and increase the risk of blindness and stroke at least four fold. Diabetes mellitus is characterized by insulin deficiency which prevents normal regulation of blood glucose levels, and which leads to hyperglycemia and ketoacidosis. The primary cause of kidney failure leading to kidney transplantation is diabetes.

Insulin, a peptide hormone, promotes glucose utilization, protein synthesis, formation and storage of neutral lipids, and the growth of some cell types. Insulin is produced by the β cells within the islets of Langerhans of the pancreas. Early-onset diabetes (10-20% of cases) is caused by an auto-immune reaction that causes complete destruction of β cells. Adult-onset diabetes has a number of causes, but in most cases the β islet cells are defective in secretion of insulin.

Insulin injection therapy, usually with porcine or bovine insulin, prevents severe hyperglycemia and ketoacidosis, but fails to completely normalize blood glucose levels. While injection therapy has been quite successful, it fails to prevent the premature vascular deterioration that is now the leading cause of morbidity among diabetics. Diabetes-related vascular deterioration, which includes both microvascular degeneration and acceleration of atherosclerosis, can eventually cause renal failure, retinal deterioration, angina pectoris, myocardial infarction, peripheral neuropathy, and arteriosclerosis.

Large scale production of human insulin has become possible with the cloning of the human insulin gene, which has begun to replace bovine and porcine insulin as the treatment of choice. Use of human insulin has eliminated some of the problems associated with other forms of insulin, including antibody-mediated insulin resistance and allergic reactions resulting from the slightly different structures of non-human insulins. Despite these advantages, treatment with human insulin does not prevent vascular deterioration.

Insulin delivery pumps have been developed which administer varying doses of insulin based on activity, diet, time of day, and other pre-programmed factors. While such devices improve blood sugar control, they also do not prevent vascular deterioration.

While the maintenance of blood sugar control with exogenous insulin has proved of significant value in reducing diabetes-related complications, the most effective form of therapy is thought to be providing patients with an endogenous source of insulin. Surgical transplantation of part or all of the pancreas is difficult, however, because the pancreas is a fragile and complicated organ, the only practical source is a deceased donor. Further, only a small portion of the pancreas, the β cells of the islet of Langerhans, produce insulin; the remainder of the pancreas presents a potent target for transplant rejection. Transplantation of just the islets of Langerhans is a desirable goal, as they continue to secrete appropriate amounts of insulin in response to nutritional signals even when isolated from the rest of the pancreas.

Islet cell transplantation has been successfully performed in animals made diabetic by prior treatment with a drug which destroys β cells. Successful transplantation in these animals has been shown to restore normal blood glucose regulation and reduce further vascular deterioration. In these animal models, it is possible to use islet cells from donors which are syngeneic (fully tissue compatible, also referred to as histocompatible) to the diabetic recipient. In humans, fully tissue compatible donors are rare (1 in 200,000) and so from a practical standpoint, islets from mismatched humans (allografts) or non-humans (xenografts) will need to be employed.

A major problem associated with transplantation of any tissue is immune-mediated graft rejection in which the recipient's T-lymphocytes recognize donor histocompatibility antigens as foreign. Thus, even though human and xenogeneic insulin can be used to partially control diabetes, the use of allografts and/or xenografts as a true therapy for diabetes depends on preventing transplant rejection. Current regimes for transplanting many tissues and organs require life-long administration of immunosuppressive drugs. These drugs have serious side-effects and can cause increased susceptibility to infection, renal failure, hypertension, and tumor development.

In addition to transplant rejection based on recognition of allogeneic and xenogeneic tissue differences, it has been observed first in diabetic rodents and later in humans that transplanted islet cells could be destroyed in diabetic hosts even when host and donor were genetically identical. Naji et al. (1981) Science 213:1390 showed that spontaneously diabetic animals maintained a skin and bone marrow allograft while an islet allograft of the same genetic makeup as the skin and bone marrow allograft was destroyed. It appears that the disease process that destroyed the native islet β cells can recur and destroy transplanted islet cells. This phenomenon, termed disease recurrence, is the process in which a target tissue is destroyed independent of histocompatibility differences between donor and host that are involved in allograft responses. This disease differs from conventional transplantation responses in several ways. Perhaps the most important difference is that the dose of immunosuppression which can be effectively used to prevent acute rejection of most allografts (e.g., kidney, liver, etc.) is not nearly as effective in preventing disease recurrence in diabetes. This lack of effectiveness is equally true for xenografts. Increasing the dose of immunosuppression leads to toxicity. Thus, it is clear that approaches must be developed which protect transplanted cells against both transplant rejection and disease recurrence.

A second problem has been the paucity of islet tissue suitable for transplantation. While sources of donor insulin from non-primate species is clinically effective in reversing hyperglycemia, xenogeneic donor tissue is subject to violent rejection. Further, the ready accessibility of non-human donors as a source of islet tissue has been to date of no practical value.

Several immunologically privileged sites in mammals allow prolonged survival of transplanted allografts (Naji & Barker (1976) J. Surg. Res. 20:261-267). The remarkable survival of islet allo- and xenografts transplanted into abdominal testes has been reported (Selawry & Fojaco (1985) Diabetes 34:1019-1024; Bellgrau & Selawry (1990) Transplantation 50:654-657; Selawry et al. (1987) Diabetes 36:1061-1067). Selawry et al. (1991) Transplantation 52:846-850 have shown that an unknown factor or factors released by testicular Sertoli cells appears to be responsible for the protection of the intratesticular islet allo- and xenografts against rejection. This unknown factor (s) has been reported to inhibit the production of IL-2 *in vitro* (Selawry et al. (1991) supra).

Selawry & Cameron (1993) Cell Transplantation 2:123-129 studied the use of Sertoli cells to establish an immunologically privileged site *in vivo* in the renal subcapsular space. Diabetic PVG rats received rat islet cells grafts with and without Sertoli cells (Sertoli enriched fraction, or SEF) and with and without cyclosporine (CsA). The results showed that 70% - 100% of the recipient rats receiving islet cells alone, islet cells and CsA alone, or islet cells and SEF alone, remained hyperglycemic. In contrast, prolonged normoglycemia in excess of 100 days was achieved in rats receiving a combination of islet cells, SEF, and CsA.

### Summary of the Invention

This invention is based in part on the discovery by the inventors that the factor produced by testicular Sertoli cells responsible for the protection of the intratesticular islet allo- and xenografts against rejection is the Fas ligand.

In accordance with one aspect of the present invention, there is provided the use of a Fas ligand for the manufacture of a medicament for suppressing T-lymphocyte-mediated transplant rejection in a recipient mammal.

In accordance with another aspect of the present invention, there is provided the use of a cell which expresses a Fas ligand for the manufacture of a medicament for suppressing T-lymphocyte-mediated graft rejection in a recipient mammal.

In accordance with another aspect of the present invention, there is provided the use of a Fas ligand, or a cell which expresses a Fas ligand, for the manufacture of a medicament for suppressing or preventing T-lymphocyte-mediated disease recurrence in a mammal.

In accordance with another aspect of the present invention, there is provided the use of a Fas ligand, or a cell which expresses the Fas ligand, for the manufacture of a medicament for treating a T-lymphocyte-mediated disease in a mammal.

In accordance with another aspect of the present invention, there is provided the use of a polynucleotide encoding a Fas ligand for the manufacture of a medicament for suppressing T-lymphocyte-mediated graft rejection in a recipient mammal.

In accordance with another aspect of the present invention, there is provided the use of a polynucleotide encoding a Fas ligand for the manufacture of a medicament for suppressing or preventing T-lymphocyte-mediated disease recurrence in a mammal.

In accordance with another aspect of the present invention, there is provided the use of a polynucleotide encoding a Fas ligand for the manufacture of a medicament for treating a T-lymphocyte-mediated disease in a mammal.

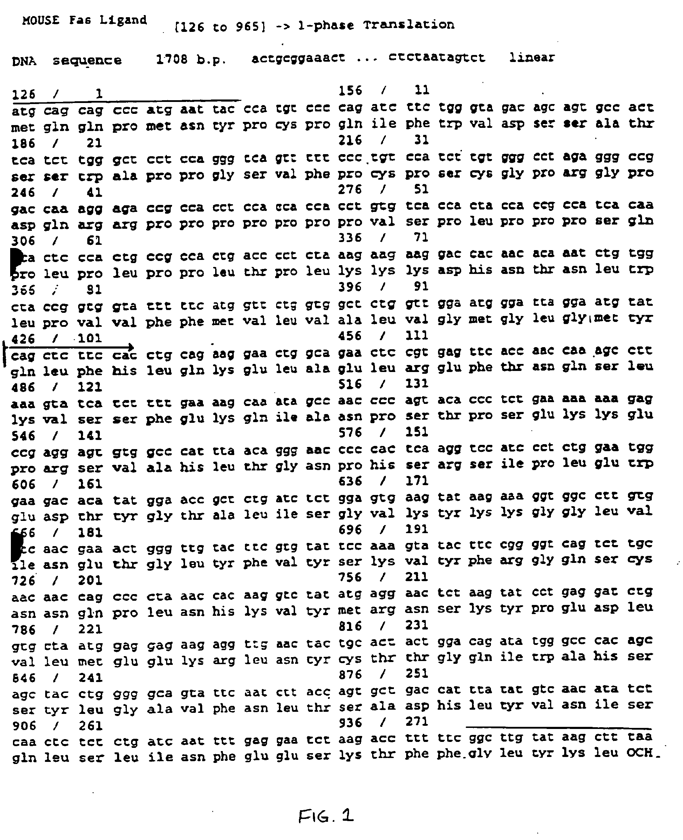

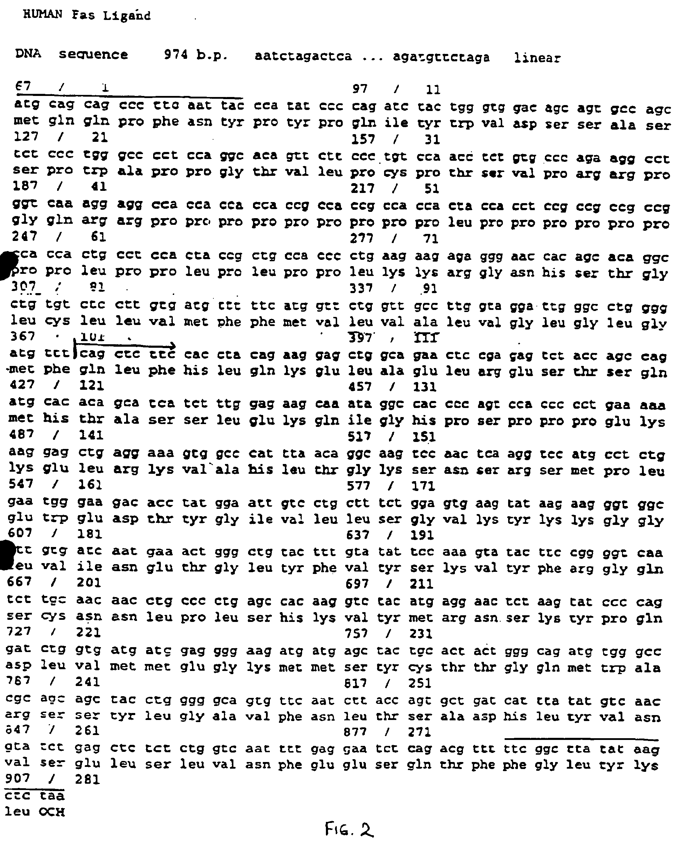

The pharmaceutical compositions useful in the treatment of transplant rejection, are comprised of a pharmaceutically acceptable excipient and a therapeutically effective amount of Fas ligand. The Fas ligand useful in the method of the invention includes intact and soluble forms of human and mouse Fas ligand. The human soluble polypeptide having the amino acid sequence between amino acid 103 to 281 as shown in Fig. 2. The invention includes the use of polynucleotide sequence encoding the human soluble polypeptide having the nucleotide sequence between nucleotides 374 to 909 (Fig. 2). Also included is mouse soluble polypeptide having the amino acid sequence between amino acid 101 to 280 as shown in Fig. 1. The invention includes the use of polynucleotide sequence encoding the mouse soluble polypeptide having the nucleotide sequence between nucleotides 426 to 966 (Fig. 1). We also describe monoclonal and polyclonal antibodies specific to the soluble Fas ligand polypeptides of the invention.

The invention features a method for suppressing lymphocyte-mediated rejection by a recipient mammal of transplanted tissue by administering to the recipient mammal an effective amount of Fas ligand. The donor mammal may be the same or a different species as the recipient mammal.

In another aspect, the invention also features a method for suppressing and preventing lymphocyte-mediated disease recurrence, such as recurrence of diabetic disease, by administering to the patient in need thereof an effective amount of Fas ligand.

In another aspect, the invention also features a method for treating lymphocyte-mediated primary disease, such as juvenile diabetes, by administering to the patient an effective amount of Fas ligand.

The invention further features a method for suppressing lymphocyte-mediated rejection by a recipient mammal of transplanted tissue by introducing into the recipient mammal a cell which expresses Fas ligand.

The invention also features a method for suppressing and preventing lymphocyte-mediated disease recurrence, such as recurrence of diabetic disease, by introducing into a mammal in need thereof a cell which expresses Fas ligand.

This invention also features a method for treating lymphocyte-mediated primary disease, such as juvenile diabetes, by introducing into a mammal in need thereof a cell which expresses Fas ligand.

We also describe a transgenic non-human animal containing a DNA sequence encoding a Fas ligand polypeptide in its germ and somatic cells. The transgenic non-human animal of the invention is capable of expressing biologically active Fas ligand in cells of all organs and tissues, and is thus useful as a source of donor organs or cells which, because they express Fas ligand, will be less susceptible to rejection.

In one embodiment of the Fas ligand administration of the invention, purified natural or recombinant Fas ligand polypeptide is provided to the recipient mammal at the site of the transplant graft. In another embodiment, the transplanted tissue itself functions as a source of Fas ligand. In this embodiment, transplanted tissue is obtained from a non-human animal which contains the gene encoding the Fas ligand in its germ and somatic cells. The transgenic tissue containing the Fas ligand gene maintains its ability to express biologically active Fas ligand when transplanted into the recipient host animal. The invention includes the transplant of Fas ligand-expressing tissue alone, e.g., transplant of transgenic islet cells into a diabetic patient, or transplantation of Fas ligand-expressing autologous tissue, e.g., fibroblasts, along with non-manipulated donor tissue, e.g., transplant of a non-transgenic islet cells to a patient in need thereof with Fas ligand-expressing tissue to the graft site, thereby creating an artificial immunologically-privileged site. In this case, the transplanted Fas ligand-expressing tissue functions to suppress rejection of the transplanted islet cells.

In another aspect, the invention provides a method of coating or perfusing cells, tissues or organs to be transplanted with a Fas ligand polypeptide such that a protective barrier against rejection by the recipient subject is formed, e.g., an immunulogically-privileged site is created.

We also describe a method of treating or preventing autoimmune inflammation by administration of Fas ligand or autologous cells expressing Fas ligand to the site of inflammation.

In yet a further aspect, the invention provides methods for inducing tolerance to either a foreign protein or a transplanted cell, tissue or organ, by administration of autologous cells co-transfected with genes encoding Fas ligand and the foreign protein of interest. In a related aspect, methods are provided for pre-tolerizing a recipient subject to transplanted cells, tissues, or organs prior to transplantation by administering allogeneic cells from the donor source which express the Fas ligand. Methods are also provided for treatment or prevention of disease by providing by gene therapy the gene encoding Fas ligand.

We also describe a method of providing immune protection for viral vectors and foreign genes used in gene therapies by inserting a polynucleotide encoding the Fas ligand into a viral vector encoding a foreign gene.

Other features and advantages of the invention will be apparent from the following description of the preferred embodiments thereof, and from the claims. Drawings

Fig. 1 is the nucleotide and amino acid sequences for mouse Fas ligand (Takahashi et al. (1994) Cell 76:969-976). The amino acid sequence of the intact protein is numbered from amino acid 1 to 280 (nucleotide sequence 126-966). The sequence of soluble Fas ligand is from amino acids 101 to 280 (nucleotide sequence 426-966). Lines denote the sequence of the primers used to synthesize the intact and soluble polypeptides.

Fig. 2 is the nucleotide and amino acid sequences for human Fas ligand (Takahashi et al. (1994) Intl. Immunol. 6:1567-1574). The amino acid sequence of the intact protein is numbered from amino acid 1 to 281 (nucleotide sequence 67-907). The sequence of soluble Fas ligand is from amino acids 103 to 280 (nucleotide sequence 374-909). Lines denote the sequence of the primers used to synthesize the intact and soluble polypeptides.

### Detailed Description

Reference will now be made in detail to useful embodiments of the invention, which, together with the following examples and claims, serve to explain the principles of the invention. It is to be understood that this invention is not limited to the specific examples described, and as such may, of course, vary. It is also to be understood that the terminology used herein is with the purpose of describing particular embodiments only, and is not intended to limit the scope of the invention which will be limited only by the appended claims.

Unless defined otherwise, all technical and scientific terms used herein have the same meaning as commonly understood by one of ordinary skill in the art to which this invention belongs. Although any methods and materials similar to or equivalent to those described herein can be used in the practice or testing of the invention, the preferred methods and materials are now described. All publications mentioned herein are incorporated herein by reference to describe and disclose specific information for which the reference was cited in connection with.

The immune system consists of a collection of bone marrow-derived cells and plasma proteins which work together to fight threats to an organism's integrity. Occasionally, the immune system actually causes disease, e.g., autoimmune disease and transplant rejection. The central cell in the immune system is the T lymphocyte. T cells have receptors which specifically recognize antigens. Upon engagement with their antigen receptor, helper T cells are activated to produce and secrete lymphokines which activate the effector arm of the immune response. Lymphokines activate macrophages and cytotoxic T cells, and stimulate B cells to secrete antibodies to the antigen. As a result of the initiation of the inflammatory response, neutrophils may also be activated. When T cells first encounter antigen, they express the Fas molecule on their surface. Fas (CD95) is a 45 kD cell surface glycoprotein and member of the tumor necrosis factor receptor superfamily (Nagata and Golstein (1995) Science 267:1449; Smith et al. (1994) Cell 76: 959). Although Fas is expressed in many tissues including liver, heart and ovaries (Watanabe-Fukunaga et al. (1992) J. Immunol. 148:1274; Leithauser et al. (1993) Lab. Invest. 69:415), its major biological role appears to be in the regulation of immune responses (Nagata and Golstein (1995) supra). *Lpr* mice which lack the ability to express functional Fas accumulate large numbers of abnormal T and B cells in their peripheral lymphoid organs and develop autoimmune disease.

Antibodies to Fas induce apoptosis in a variety of cell lines derived from lymphoid leukemias (Traugh et al. (1989) Science 245:301; Yonehara et al. (1989) J. Exp. Med. 169:1747; Debatin et al. (1990) Lancet 335:497). Resting lymphocytes express low levels of Fas and are not killed by anti-Fas antibodies (Miyawaki et al. (1992) J. Immunol. 149:3753; Daniel & Krammer (1994) J. Immunol. 152:5624; Alderson et al. (1995) J. Exp. Med. 181:71). However, within 24 hours following antigen stimulation, T and B cells upregulate Fas expression, and after an additional 2-3 days, the Fas-expressing T cells die if Fas is crosslinked (Miyawaki et al. (1992) J. Immunol. 149:3753; Owen-Schaub et al. (1992) Cell Immunol. 140:197). The crosslinking of Fas and killing of activated lymphocytes is mediated *in vivo* by the Fas ligand, a 40 kD, type II membrane glycoprotein related to tumor necrosis factor (Nagata and Golstein (1995) supra; Rouvier et al. (1993) J. Exp. Med. 177:195).

The human and mouse Fas ligand genes have been isolated and sequenced (Takahashi et al. (1994a) Intl. Immunol. 6:1567-1574; Takahashi et al. (1994b) Cell 76:969-976). The purified Fas ligand exhibits cytolytic activity against cells expressing Fas. Experiments show that Fas ligand expression occurs coordinately with Fas upregulation during an immune response.

The invention includes the use of intact and soluble Fas ligand polypeptides. The intact Fas ligand is a membrane-associated polypeptide having the immunosuppressive activity described herein. The soluble Fas ligand polypeptide consists of the extracellular carboxy-terminal portion of the Fas ligand polypeptide and possesses the immunosuppressive activity of the native protein. The soluble Fas ligand may be complexed with one or more molecules, including Fas ligand dimers or trimers, in order to optimize biological activity. Such complexes may be formed by various methods known to the art, including chemical modification and covalent crosslinking. The Fas ligand is used, according to the invention, to suppress lymphocyte-mediated rejection of transplanted tissue. The Fas ligand is also used to prevent lymphocyte-mediated disease recurrence, and to treat lymphocyte-mediated primary disease. The methods of the invention involve providing an amount of Fas ligand effective to suppress lymphocyte-mediated rejection of transplanted tissue, disease recurrence, and/or primary disease.

During a lymphocyte-mediated immune response, helper T cells first become activated and provide factors (e.g., lymphokines) which serve to activate the effector cells of the immune system. These cells include the helper T cells themselves, but also include cytotoxic T lymphocytes, B lymphocytes, monocytes/macrophages, and neutrophils. All of these leukocytes become activated during a T-lymphocyte-mediated immune response in order to carry out the effector arm of immunity (e.g., erradication of bacteria, neutralization of toxins, destruction of virally infected cells or transplanted cells, etc.). When they are activated, each of these cell populations express high levels of functional Fas on their surface. Thus, all of these cells can serve as targets of the Fas-ligand mediated immunosuppression methods of the invention.

In rejection of an allograft, the immune system of the recipient animal has not previously been primed to respond because the immune system for the most part is only primed by environmental antigens. Tissues from other members of the same species have not been presented in the same way that, for example viruses and bacteria have been presented. In the case of allograft rejection, immunosuppressive regimens are designed to prevent the immune system from reaching the effector stage. However, the immune profile of xenograft rejection may resemble disease recurrence more than allograft rejection. In the case of disease recurrence, the immune system has already been activated, as evidenced by destruction of the native islet cells. Therefore, in disease recurrence the immune system is already at the effector stage. The Fas ligand is able to suppress the immune response to both allografts and xenografts because lymphocytes activated and differentiated into effector cells express Fas, and thereby are susceptible to the Fas ligand.

A reaction or disease is considered to be lymphocyte-mediated when T-lymphocytes are required in mediating the reaction or disease effect. Where cells of the tissue for transplantation (the "donor" tissue) bear on their surfaces foreign histocompatibility antigens, these antigens cause cytotoxic T-lymphocyte activation in recipients, terminating in donor cell destruction after several sequential activation steps. The cascade is initiated by conjugate formation between the antigen-specific T-cell receptor on host T-lymphocytes and the major histocompatibility antigens on the donor cell. Conjugate formation is followed by T-lymphocyte-mediated activation, resulting in donor cell death. This process can eventually result in rejection even in intra-species transplantation. According to the invention, this problem is addressed by suppressing the T-lymphocyte response prior to the stage where donor cell destruction is initiated.

While the invention specifically addresses transplant rejection and disease recurrence, the invention is useful for inhibiting other types of lymphocyte-mediated medical conditions. Lymphocytes activated by disease as well as by the introduction of foreign grafts express Fas, and therefore, are susceptible to treatment with Fas ligand. In general terms, the Fas ligand as an immunosuppressive agent is most active against a primed or activated immune system. The primed or activated immune system may be associated with disease conditions in which either T-lymphocytes or B-lymphocytes are activated. Activated T-lymphocytes are associated with disease in graft versus host reactions (e.g., bone marrow transplantation) and most forms of autoimmunity, including but not restricted to, multiple sclerosis, rheumatoid arthritis, lupus, and myasthenia gravis. Fas expressing leukemia may also be susceptible to treatment with the Fas ligand, since Fas is expressed by B- and T-lymphocyte tumors.

The Fas ligand may be used to treat chronic transplant rejection. It is recognized by the art that most transplants undergo a chronic graft destructive process. The mechanism of chronic transplant rejection differs from conventional allograft immunity and conventional immunosuppression has been ineffective in its treatment. Chronic graft rejection may be treated with the Fas ligand, resulting in successful engraftment for longer periods and allowing donor tissue to be used for new recipients.

The Fas ligand may also be used to treat acute graft rejection. Often a transplant recipient host goes through a crisis period where the transplant undergoes a severe graft rejection which cannot be resolved with the immunotherapy (usually cyclosporine) that may prevent the immune system from reaching the effector phase but is not effective against activated effector cells. A conventional route of therapy for acute graft rejection is the use of antibody to the T-lymphocyte receptor complex. This results in severe immunosuppression in the recipient host. Treatment with the Fas ligand should provide a more specific treatment for activated cells, that is, for cells attacking the transplant tissue, not all the T-lymphocytes present in the immune system.

The term "treatment", "treating" and the like are used herein to generally mean obtaining a desired pharmacologic and/or physiologic effect. The effect may be prophylactic in terms of completely or partially preventing a disease, disorder or symptom thereof and/or may be therapeutic in terms of a partial or complete cure for a disease and/or adverse effect attributable to the disease. The term "disease" is broadly defined to include immune-mediated rejection of transplanted cells, tissues or organs by the recipient subject, as well as diabetes, rheumatoid arthritis, multiple sclerosis, cystic fibrosis, etc., resulting from a immune reaction mediated by Fas-expressing cells.

More specifically, "treatment" is intended to mean providing a therapeutically detectable and beneficial effect on a patient suffering from or at risk from acute or chronic rejection of transplanted cells, tissues or organs, T-lymphocyte-mediated disease recurrence, T-cell-mediated primary disease, and T-cell-mediated autoimmune disease.

Still more specifically, "treatment" shall mean preventing, alleviating, and/or inhibiting (1) immune-mediated rejection of transplanted cells, tissues or organs mediated by T lymphocytes, (2) immune-mediated disease recurrence, such as recurrence of diabetic disease and (3) immune-mediated primary disease, such as juvenile diabetes, rheumatoid arthritis, cystic fibrosis, or multiple sclerosis. The method of the invention specifically targets Fas-expressing immune effector cells involved in the immune response, and may be used to treat undesirable autoimmune reactions such as allergies.

By the term "Fas ligand" or "Fas ligand polypeptide" is meant the entire amino acid sequence of the Fas ligand polypeptide or a fragment thereof, having an immunosuppressive effect, and reacting with antibodies specific for the Fas ligand polypeptide. The Fas ligand includes membrane-associated (intact) and soluble Fas ligand polypeptides, and the methods of the invention include the use of intact and soluble Fas ligand in the treatment of lymphocyte-mediated diseases and disorders. By the term "soluble Fas ligand" as used herein is meant fragments of the Fas ligand containing the extracellular carboxy-terminal portion of the Fas ligand polypeptide possessing the immunosuppressive activity of the native Fas ligand, and able to react with antibodies specific for Fas ligand. As described below, intact or soluble Fas ligand is characterized by its ability to create an immunologically-privileged site in a mammalian body, thereby protecting transplanted tissue from rejection by the recipient subject. The soluble Fas ligand may be complexed with one or more molecules, including Fas ligand dimers or trimers, in order to optimize in vivo biological activity. Such complexes may be formed by ionic interactions, hydrogen bonding, or covalent bond formation between molecules, including crosslinking. Fas ligand complexes may be formed between Fas ligand molecules or between one or more Fas ligand molecules and other molecules, including peptides, proteins, lipids, and/or carbohydrates. The methods of the invention include the use of intact or soluble Fas ligand from any mammalian source which is effective in suppressing immune responses mediated by Fas-expressing effector cells.

By the term "effective amount" or "therapeutically effective amount" and the like is meant an amount sufficient to prevent or inhibit the disease being treated. The exact size of an effective amount of the Fas ligand to be administered according to the method of the invention will depend on a number of factors, including the particular recipient and severity of the condition being treated. For example, an effective amount of Fas ligand is the amount effective to inhibit T-lymphocyte-mediated transplant rejection and disease recurrence.

There is in the art recognized need for a safe and effective new form of immunosuppressive agent - one that would resist the attacks of the immune response in disease recurrence as well as the violent immune response to xenogeneic donor tissue. This invention addresses that need by providing methods which allow the use of non-human tissue for transplantation into a human patient in need thereof. The method of the invention prevents rejection of xenogeneic tissue. The invention thus permits not just intra-species transplantation of tissues and organs, but xenografts as well, opening up the possibility of "farming" of donor organs and tissues in non-human mammals for transplantation into human patients. In the case of xenografts, this invention may be practiced along with other methods for masking, modifying, or eliminating undesirable antigens on the surface of donor cells, such as the method described in U. S. Patent No. 5,283,058, herein incorporated by reference.

By "endogenous source of Fas ligand" is meant providing means by which the Fas ligand is synthesized and secreted within the host recipient mammal itself. In contrast, an exogenous source of Fas ligand may be provided by continuous infusion of purified Fas ligand through a pump implanted into the host recipient mammal.

Donor Tissue. Donor tissue may be obtained from the same or a different species as the recipient mammal. The term "donor tissue" includes cells and organs from a donor mammal, including but not limited to islet cells, kidney, heart, liver, lung, brain, and muscle tissue.

The donor tissue may be obtained from any mammal, preferably pigs. Pigs offer many advantages for use as organ and cell donor animals. For example, many porcine organs, such as the heart and kidney, are of a similar size to human organs.

Purified Fas Ligand. The Fas ligand administered may be obtained from naturally occurring sources, and may be purified from any animal or cell source, including mouse, rat, pig, etc. A simple method for purifying Fas ligand from cultured PC60-d10S cells is described by Suda & Nagata (1994) J. Exp. Med. 179:873.

The invention also contemplates use of purified Fas ligand produced by recombinant DNA technology. The recombinant methods necessary to produce high quantities of Fas ligand of high purity are described below for production of intact and soluble mouse and human Fas ligand polypeptides.

Administration of Fas ligand. Purified Fas ligand is administered to a transplant recipient to suppress T-lymphocyte-mediated rejection of the transplanted tissue by the recipient mammal and to suppress recurrence of a disease which destroyed the endogenous tissue being replaced. Purified Fas ligand may be administered by a number of methods known in the art. In one embodiment of the invention, a therapeutic or pharmaceutical composition comprising Fas ligand is administered in an effective amount to a mammal sufficient to prevent a lymphocyte-mediated transplant rejection or disease recurrence. The therapeutic or pharmaceutical composition of the invention may be administered in a variety of ways, including by injection or by continuous infusion from an implanted pump. Other appropriate administration forms are envisioned. For example, semipermeable implantable membrane devices that are useful as means for delivering drugs or medications are known. The encapsulation of cells that secrete neurotransmitter factors, and the implantation of such devices into the brain of patients suffering from Parkinson's disease has been described. See, U. S. Patent No. 4,892,538; U. S. Patent No. 5,011,472; U. S. Patent No. 5,106,627. Formulations of purified Fas ligand may contain one or more agents and pharmaceutically acceptable excipients. By the term "excipient" or "pharmaceutically acceptable excipient" is meant a carrier which is acceptable in the sense of being compatible with other ingredients of a pharmaceutical composition and not deleterious to the recipient.

In one embodiment of the invention, Fas ligand is therapeutically administered by implanting into patients, transfected cells capable of expressing and secreting a biologically-active form of Fas ligand.

Example 1 describes the transplantation of rat islet cells into the renal subcapsular space of diabetic PVG rats. Pumps dispensing saline (controls) or purified Fas ligand (experimentals) are implanted in proximity to the graft site.

An alternative to administration of purified Fas ligand to the graft site, transplant tissue can be grown in transgenic animals which have been genetically altered to contain the Fas ligand gene sequence. Such transgenic animals can be made by standard transgenic techniques (Example 2). Example 3 describes transplantation of islet cells from transgenic rats wherein the transplanted tissue itself is an endogenous source of Fas ligand.

The invention may be used to treat a number of human disease conditions resulting from destruction of endogenous cells, such as the destruction of insulin producing pancreatic islet β cells in diabetes. An important feature of the invention is that it makes possible use of non-human mammals as tissue and organ donors for human patients. The above methods describe the use of the invention to treat diabetic human patients by transplantation of xenogeneic islet cells. The xenogeneic islet cells may be obtained from, for example, normal or transgenic pigs expressing the Fas ligand polypeptide. Example 4 describes transplantation of transgenic porcine islet cells into a diabetic human patient.

The production of Fas ligand mRNA in Sertoli cells was established as described in Example 6. mRNA from purified rat Sertoli cells probed with a Fas ligand specific oligonucleotide showed that Sertoli cells are the primary if not exclusive source of the Fas ligand in testicular tissue. To test whether the absence of a functional Fas ligand molecule prohibited Sertoli cells from providing their immunosuppressive function, testicular tissue was transplanted a into BALB/c mice (Example 7). Both B6 and B6-*gld* donors are genetically incompatible with the BALB/c recipients. The B6-*gld* strain carries a point mutation in the Fas ligand gene preventing the expressed protein from functioning to engage apoptosis. While grafted B6 tissue remained healthy in the recipient BALB/c animals, the grafted B6-*gld* tissue was completely destroyed within 7 days. These experiments were repeated with transplanted Sertoli cells isolated from B6 and B6-*gld* animals into BALB/c recipients (Example 8). The results were identical to those obtained with transplanted tissue (Example 7). Table 1 summarizes the results observed for allograft transplantation of intact testis tissue and isolated Sertoli cells derived from B6, B6-*gld*, and B6-*lpr* mice into BALB/c recipients. Example 10 describes experiments in which BALB/c recipient mice transplanted with intact testis tissue or isolated Sertoli cells derived from B6 mice were challenged with or without B6 spleen cells 14 days after transplantation. The results show that Fas ligand bearing cells and tissue resisted allograft rejection even when the host destroyed other non-Fas ligand bearing allogeneic tissue (Table 2).

The genes for membrane-associated (intact) and soluble mouse and human Fas ligand were isolated and cloned as described in Example 11. The DNA sequence encoding soluble mouse Fas ligand polypeptide is shown in Fig. 1, encoded by the sequence between the nucleotides from position 426 to 966, and amino acids 101 to 280 (Takahashi et al. (1994) Cell 76:969-976). The DNA sequence encoding soluble human Fas ligand polypeptide is shown in Fig. 2, encoded by the sequence between the nucleotides from position 374 to 909, and amino acids 103 to 281 (Takahashi et al. (1994) Intl. Immunol. 6:1567-1574).

Purified intact and soluble polypeptides were used for generation of monoclonal and polyclonal antibodies to each polypeptide (Example 12). The antibodies of the invention include intact molecules as well as fragments thereof,- such as Fab, F(ab')₂, and Fv, which are capable of binding the epitopic determinant. Therapeutic uses of the Fas ligand and gene therapy are described in Examples 13 and 14.

All of the invention methods and compositions employ soluble Fas ligand or an antigenic fragment thereof.

The following examples are intended to illustrate but not limit the invention. While they are typical of those that might be used, other procedures known to those skilled in the art may alternatively be used.

### Example 1. Suppression of T-lymphocyte-Mediated Rejection of Transplanted Tissue by Administration of Fas Ligand.

Islet preparation. Freshly isolated islet cells from a rat are prepared according to known methods. See, for example, London et al. (1990) Transplantation 49:1109-1113. Under appropriate circumstances, islet cells may be pretreated prior to transplantation to conceal ("mask") donor antigens or modify graft immunogenicity by methods known in the art, for example, those described in U. S. Patent No. 5,283,058,

Purified Fas ligand. Purified Fas ligand may be obtained from a mammalian source or produced in vitro as a recombinant protein. In one embodiment of the invention, purified Fas ligand is obtained from a naturally occurring source. A simple method for large scale purification of Fas ligand from cultured cells has been reported (Suda & Nagata (1994) supra). Briefly, cells expressing Fas ligand are cultured and harvested. A solubilized membrane fraction is purified by affinity purification, and the Fas ligand eluted as described by Suda & Nagata (1994) supra.

Bioassay of Fas ligand. The biological activity of purified Fas ligand is assessed in vitro with the cytotoxicity assay described by Suda & Nagata (1994) supra.

Transplantation of rats and administration of purified Fas ligand . Diabetic PVG rats are grafted with islet cells and implanted with pumps dispensing saline (controls) or purified Fas ligand (experimental) as follows. Diabetic PVG rats are anesthetized with methoxyflurane USP and the left flank opened to expose the kidney. Islets cells (10 islets/g of body weight) are injected under a renal capsule as described by Selawry & Cameron (1993) supra. A pump programmed to dispense either saline or purified Fas ligand over an empirically-determined period of time is implanted under the renal capsule. Cyclosporine (CsA) may be injected subcutaneously 25 mg/kg per day for a seven day period.

Recipient rats are evaluated for plasma glucose levels. Urine volumes and urine glucose contents are obtained and determined as described (Selawry & Cameron (1993) supra). Recipient rats receiving Fas ligand become normoglycemic over a prolonged period of time.

### Example 2. Production of Transgenic Mammals Containing DNA Encoding the Fas Ligand.

A transgenic rat whose germ cells and somatic cells contain the Fas ligand gene is produced by methods known in the art. See, for example, U. S. Patent No. 4,736,866 describing production of a transgenic mammal, herein incorporated by reference. Generally, the DNA sequence encoding the Fas ligand is introduced into the animal, or an ancestor of the animal, at an embryonic stage (preferably the one-cell, or fertilized oocyte, stage, and generally not later than about the 8-cell stage). There are several methods known to the art of introducing a foreign gene into an animal embryo to achieve stable expression of the foreign gene. One method is to transfect the embryo with the gene as it occurs naturally, and select transgenic animals in which the foreign gene has integrated into the chromosome at a locus which results in its expression. Other methods involve modifying the foreign gene or its control sequences prior to introduction into the embryo. For example, the Fas ligand gene may be modified with an enhanced, inducible, or tissue-specific promoter.

Tissues of transgenic rats are analyzed for the presence of Fas ligand, either by directly analyzing RNA, by assaying the tissue for Fas ligand, or by assaying conditioned medium for the secreted Fas ligand. For example, cells obtained from the transgenic rat are cultured in the presence of ³⁵S-methionine, the supernatant subjected to immunoprecipitation with antibodies to Fas ligand. Precipitated proteins are resolved by reducing SDS-polyacrylamide gel electrophoresis, and visualized by autoradiography. Conditioned medium may also be tested for *in vitro* cytotoxic activity by the method of Suda & Nagata (1994) supra.

### Example 3. Transplantation of Transgenic Islet Cells Expressing the Fas Ligand.

Islet cells are obtained from the transgenic rat of Example 2 and grafted into diabetic PVG rats by the methods described in Example 1. Recipient rats, evaluated as described above, achieve normoglycemia for prolonged periods of time.

### Example 4. Transplantation of Transgenic Porcine Islet Cells Into a Human Diabetic Patient.

A transgenic pig is obtained all of whose germ cells and somatic cells contain a recombinant DNA sequence encoding human Fas ligand. The human Fas ligand DNA sequence was introduced into the pig by methods known to the art. Islet cells are obtained from the transgenic pig by the methods described in Example 2 and are grafted into diabetic human patient by methods known in the art. The human patient, evaluated appropriately, achieves normoglycemia for prolonged periods of time.

### Example 5. Other Embodiments.

The method of the invention may also be used to prevent a recurring disease which resulted in destruction of endogenous tissue. For example, disease recurrence mediated by T-lymphocytes directed to islet β cell antigens results in destruction of grafted islet cells. Therefore, providing Fas ligand to the graft site prevents recurrence of diabetes and allows normoglycemia to be achieved in recipient mammals by suppressing the immune response directed to islet β cell antigens.

### Example 6. Production of Fas Ligand mRNA by Isolated Sertoli Cells.

cDNA synthesis. Total RNA from purified rat Sertoli cells was isolated from cell pellets by the method of Chomczynski and Sacchi (1987) Anal. Biochem. 162:156. The RNA (5 *µ*g) was first denatured in methyl mercuric hydroxide (10 mM final concentration)(Alfa Products, Ward Hill, MA) and converted to cDNA in Taq (*Thermus aquaticus*) DNA polymerase buffer (50 mM KC1, 10 mM Tris-HCl, pH 8.3, 2.5 mM MgCl₂, and 0.01% gelatin) in the presence of RNA guard (20 units)(Pharmacia, Piscataway, NJ), β-mercaptoethanol (40 mM), dNTPs (0.5 mM)(Pharmacia), 1 µg random hexamers (Pharmacia) and AMV (Avian myeloblastosis virus) reverse transcriptase (20 units)(Life Sciences Inc., St. Petersburg, FL) in a 50 µl reaction for 90 min at 42°C.

PCR amplification. Following synthesis, 5 µl of the cDNA was transferred to a tube on ice containing 200 mM dNTPs (Pharmacia), Taq polymerase buffer containing 1.5 mM MgCl₂, Taq DNA polymerase (1 unit) (Perkin Elmer Cetus, Norwalk, CT) and the rat Fas ligand specific oligonucleotide primers 5'-GCCCGTGAATTACCCATGTC-3' (SEQ ID NO:1) and 5'-TGGTCAGCAACGGTAAGATT-3' (SEQ ID NO:2) (forward and reverse, respectively). The samples were overlaid with light mineral oil (Sigma Chemical Corp., St. Louis, MO) and transferred to a thermal cycler (MJ Research, Inc., Watertown, MA). Following heating to 94°C for 5 min to denature DNA/RNA complexes, the samples were amplified for 28 cycles of 1 min at 94°C, 1.5 min at 55°C, and 2 min at 72°C, followed by a final 10 min extension at 72°C.

Detection. 20 *µ*l of the starting 50 µl reaction was separated by electrophoresis through a 1.6% agarose gel. The following samples were run: mRNA from Sertoli cells incubated at 32°C (lane 1) or at 37°C (lane 3); mRNA from Sertoli cells from a second animal incubated at 32°C (lane 2) or at 37°C (lane 4). The DNA in the gel was then transferred to nitrocellulose filters (Schleicher & Schuell, Keene, NH) according to the method of Southern (1975) J. Mol. Biol. 98:503. The filters were UV cross linked (Stratagene, San Diego, CA) and hybridized at 37°C overnight in a solution containing 6X SSC (1X SSC = 0.15 M sodium chloride and 0.015 M sodium citrate), 1X Denharts (0.02% each Ficoll 400, bovine serum albumin, and polyvinylpyrrolidone), 20 *µ*g/ml wheat germ tRNA, 0.1% SDS and 0.05% sodium pyrophosphate plus the ³²P end-labelled Fas ligand specific oligonucleotide 5'-AACATAGAGCTGTGGCACC-3' (SEQ ID NO:3). After extensive washing in 6X SSC plus 0.05% sodium pyrophosphate at 47°C, the filters were dried and exposed to Kodak X-Omat film.

An autoradiograph of the amplified rat Sertoli cell mRNA was obtained. Lanes 1 and 3 are mRNA from cells incubated at 32°C or 37°C, respectively; lanes 2 and 4 are mRNA from Sertoli cells taken from a second animal cultured at 32°C or 37°C, respectively. These results show that Sertoli cells are the dominant, if not exclusive, source of Fas ligand in the testis.

DNA sequencing. The PCR product was determined to be identical to that published by Suda et al. (1993) Cell 75:1169 by standard DNA sequencing methodology of Sanger et al. (1977) Proc. Natl. Acad. Sci. USA 78:5453.

### Example 7. Effect of Fas Ligand on Grafted Testicular Survival.

To test whether the absence of a functional Fas ligand molecule prohibited Sertoli cells from providing their immunosuppressive function, we transplanted testicular tissue from male B6-*gld* or genetically compatible but Fas ligand operative C57BL/6 (B6) mice into BALB/c recipients. The B6-*gld* and B6 strains are identical at the major histocompatibility complex (MHC) and also share essentially all other (minor) histocompatibility antigens with the exception of the Fas ligand. B6-*gld* carries a point mutation in the Fas ligand gene (Takahashi et al. (1994b) supra). B6-*gld* and B6 differ from the BALB/c strain at both the MHC and multiple minor loci.

Testicular grafts of B6*-gld* or B6 tissue were transplanted under the kidney capsule of BALB/c recipients. A mouse is weighed and injected with Avertin (12 ml/g), and anesthetized with Metophane. The mouse is shaved under the rib cage on the left side, and a small incision (about 8 mm) is made through the body wall. The adipose tissue attached to the posterior end of the kidney is pulled such that the kidney is externalized. The kidney is kept moist with Hank's Basic Salt Solution (HBSS). A straight incision is made on the posterior end of the kidney and the kidney capsule carefully loosened from the kidney with a probe. The tissue to be transplanted is deposited under the capsule and gently moved to the anterior part of the kidney with a probe. The kidney is replaced inside the body and the incision closed.

Blood Clot Transplantation Procedure. This procedure is designed to permit groups of cells or non-clustered islets to be transplanted under the recipient host kidney capsule. Embedding the cells within the clot allows the transplanter to place them in a relatively defined position from which they will not move.

Cells to be transplanted are transferred to a siliconized 15 ml centrifuge tube and allowed to settle for 5 min. Cells in a cell suspension are transferred to a siliconized 15 ml centrifuge tube and centrifuged at 300 x g for 5 min. Most of the supernatant is removed and the cells resuspended in the remaining 200 µl medium. The cells are resuspended and transferred to a 300 µl microfuge tube, centrifuged at 500 - 1000 x g for 30 sec and placed on ice. Most of the supernatant is removed, leaving about 4-5 mm fluid. With a scalpel, the top is cut just above the fluid level, and the remaining fluid removed with a capillary tube. Blood is drawn from the tail vein of the recipient animal and approximately 5 µl blood added to the cell pellet. A clot is allowed to form for 10 min. Residual sera is drawn off. The cells are embedded within the clot matrix and are not easily dislodged. The cell clot may then be transplanted into the kidney capsule.

Results. On days 2 and 7 (B6-*gld*) or days 2, 7, and 28 (B6), the grafted tissue was analyzed macroscopically and microscopically for graft rejection. A recipient BALB/c mouse was euthanized with an overdose of penthrane. The kidney containing the graft was removed, fixed in a formal saline buffer solution and processed by routine histologic techniques. The kidney was embedded in paraffin after which 5 µl sections were cut and stained with hematoxylin and eosin. Renal tissue obtained from BALB/c kidney engrafted with B6 tissue appeared structurally normal by light microscopy. Transplanted tissue was observed adjacent to the kidney capsule. It appeared no different in morphology from that which was observed when histocompatible-genetically identical BALB/c tissue was used as the source of donor tissue. In B6*-gld* engrafted kidney there was extensive infiltration of lymphocytes in the graft by day 2, and the architecture of the testis tissue was disrupted. The renal tissue also showed obvious lymphocytic infiltration adjacent to as well as within the graft. By day 7, there was little recognizable testis tissue and lymphocytic infiltrate was diminished, indicating that the destructive process had peaked before this time. These findings establish the role of the Fas ligand in immunosuppression, and show that the presence of a functional Fas ligand gene protects transplanted testicular tissue from graft rejection.

### Example 8. Effect of Fas Ligand on Sertoli Cell immunosuppressive Activity.

To establish if isolated Sertoli cells could duplicate the results obtained with testis tissue grafts (Example 6), Sertoli cells were isolated and purified from testicular tissue of B6-*gld* and B6 mice and transplanted as single cell suspensions under the kidney capsule in BALB/c mice, essentially as described by Selawry and Cameron (1993) Cell Transplantation 2:123 and Example 7 above. Testis were removed from mice and cut into small pieces in 5 ml HAM'S F12/DMEM media (Ham's media). The tissue was place in a 50 ml tube, 25 mls Ham's media added, and pelleted by centrifugation at 800 x g for 2-5 min. The pellet was resuspended in 20 ml Ham's media containing 20 mg trypsin and 0.4 mg DNAse. The resulting mixture was placed in a 250 ml flask in a shaking water bath at 37°C for 30 min, and pelleted at 800 x g for 2-5 min. The cell pellet was resuspended at room temperature for 10 min in 20 ml of a solution containing 1 M glycine, 2 mM EDTA, 0.01% soy bean trypsin inhibitor, and 0.4 mg DNAse. The mixture was centrifuged as above, and the cell pellet washed twice. Cells were resuspended in 20 ml Ham's media containing 10 mg collagenase, and placed in a shaking water bath at 37°C for 5 minutes, pelleted, and resuspended in 20 ml Ham's media containing 20 mg collagenase and 0.1 mg DNAse. The sample was transferred to a 250 ml flask placed in a rocking water bath at 37°C for 30 min. The cells were pelleted and washed as described above. Cells were resuspended in 10 ml Ham's media containing 20 mg hyaluronidase and 0.1 mg DNAse, and placed in 250 ml flask in a rocking water bath at 37°C for 30 min. Cells were pelleted and washed. The final pellet was kept on ice until transplanted under the kidney capsule. The pellet may be clotted with blood drawn from the host mouse (see blood clot transplantation procedure described above).

Results identical to those described in Example 7 were obtained. B6-*gld* Sertoli cells transplanted under the kidney capsule of histoincompatible BALB/c recipient mice remained intact. These results establish that the Fas ligand is an effective immunosuppressive factor responsible for the immunosuppressive effects of Sertoli cells.

Table 1 summarizes the results obtained for transplanted intact testis tissue and isolated Sertoli cells. Acceptance indicates that donor tissue in the graft had a normal morphology and no evidence of infiltration by recipient leukocytes 8 to 21 days after transplantation. Rejection indicates that there was not detectable donor tissue in the graft present but there was evidence of heavy infiltration of the graft site by recipient leukocytes.

### Example 9. Diagnostic Use of Fas Ligand Expression for Selecting Donor Tissue or Recipient Transplantation Site.

The discovery of the relationship between a functioning Fas ligand gene and protection from graft rejection may be applied diagnostically. The ability of various non-lymphoid tissue sources to express Fas ligand, detected either by examination of tissue with monoclonal antibodies to Fas ligand or by assessing Fas ligand mRNA by RT-PCR, allows prediction of the capacity for a specific tissue to be retained or rejected following transplantation. Tissues expressing a high level of Fas ligand provide a preferred site for successful organ engraftment. Screening donor tissue for Fas ligand expression will also aid in predicting transplantation success.

### Example 10. Maintenance of Fas-ligand Expressing Tissue in Allogeneic Animals Immunized Against Donor Tissue Transplantation Antigens After Transplantation.

Intact testis tissue and isolated Sertoli cells derived from B6 mice were transplanted under the kidney capsule of allogeneic BALB/c mice as described above. Fourteen days after grafting, recipient mice were injected intraperitoneally with 1 x 10⁶ donor (B6) spleen cells. Twenty-one days after transplantation, the animals were sacrificed and kidneys subjected to histological examination.

The results are shown in Table 2. Acceptance indicates that donor tissue in the graft had a normal morphology and there was no evidence of recipient leukocytes. Rejection indicates that no detectable donor tissue in the graft was present or there was heavy infiltration of recipient leukocytes at the graft site. The results show that Fas ligand-bearing testis tissue and Sertoli cells resist allograft rejection even when the host is destroying other allogeneic tissue of non-Sertoli origin.

### Example 11. Cloning and Expression of Genes Encoding Intact and Soluble Mouse and Human Fas Ligand.

The gene encoding mouse Fas ligand was cloned by reverse transcription (RT) and PCR techniques from RNA purified from the cytotoxic mouse T-cell clone DAB-6. DAB-6 cells (Duke (1989) J. Exp. Med. 170:59-71) express the Fas ligand after treatment with phorbol myristic acid and ionomycin (Rouver et al. (1993) J. Exp. Med. 177:195-200). The primers used in the synthesis of the full-length Fas ligand gene were 5' primer: 5'-CGGGATCCATGCAGCAGCCCATGAATTAC-3' (SEQ ID NO:4), and 3' primer: 5'-CGGAATTCCTTTTAAAGCTTATATAAGCC-3' (SEQ ID NO:5).

The human Fas ligand gene was cloned using the same techniques from RNA purified from peripheral blood human T-cells from healthy adults, after T-cell stimulation with concanavalin A, human IL-2, phorbol myristic acetate, and ionomycin as previously described (Takahashi et al. (1994a) supra). The primers used in the synthesis of the full-length Fas ligand gene were 5' primer: 5'-CGGGATCCATGCAGCAGCCCTTCAATTA-3' (SEQ ID NO:6), and 3' primer: 5'-CGGAATTCCTCTTAGAGCTTATATAAGCC-3' (SEQ ID NO:7).

The soluble human Fas ligand gene was synthesized with the use of the 5' primer: 5'-CGGGATCCATGCAGCTCTTCCACCTACAGAAGGAGCTGGC-3' (SEQ ID NO:8), and 3' primer: 5'-CGGAATTCCTCTTAGAGCTTATATAAGCC-3' (SEQ ID NO:9). The soluble mouse Fas ligand gene was synthesized with use of the 5' primer: 5'-CGGGATCCATGCAGCTCTTCCACCTGCAGAAGGAACTGGC-3' (SEQ ID NO:10), and 3' primer: 5'-CGGAATTCCTTTTAAAGCTTATATAAGCC-3' (SEQ ID NO:5). The internal region of the Fas ligand genes encodes the extracellular, carboxyterminal soluble portion of the Fas ligand polypeptide. This portion of the gene was engineered in frame to a signal sequence before subcloning into pCDNA3 for expression of the soluble ligand. The heterologous signal sequence is required for directing soluble Fas ligand into the secretory pathway prior to secretion from the cell.

Total RNA was isolated using the TRIzol reagent (Life Technologies, Inc.), and poly-A⁺RNA was enriched with the polyAttract kit and magnetic beads technique (Promega). The RT reaction was primed with specific 3'oligonucleotides from the published Fas ligand sequence (Takahashi et al. (1994b) supra), using Superscript II reverse transcriptase (BRL) at 50°C. The PCR reaction was performed with a mixture of Vent and Taq polymerases to ensure fidelity of the polymerase reaction. The PCR products were subcloned into a bacterial plasmid, and the products were confirmed by nested PCR reactions with internal oligonucleotides.

The cloned full-length mouse, human, and soluble human Fas ligand genes were subcloned into vectors under the control of the CMV promoters, such as pCDNA3 (Invitrogen), for expression into mammalian cells, including mouse tumor cells (L1210.3-leukemia and B16-melanoma), monkey kidney cells (COS-1), and mouse fibroblasts (BC10ME and BALB 3T3).

### Example 12. Generation of Antibodies.

To generate monoclonal and polyclonal antibodies against the intact and soluble mouse and human Fas ligands, the appropriate Fas ligand gene was subcloned in frame into the pGEX vectors as bacterial glutathione-S-transferase (GST) fusion proteins. The bacterially expressed GST-Fas fusion protein was purified on glutathione-coupled Sepharose, then eluted with reduced glutathione to yield the purified fusion protein for immunization. Monoclonal and polyclonal antibodies are made by methods known in the art (See, for example, Kohler et al. (1975) Nature 256:495).

### Example 13. Therapeutic Uses of Fas Ligand.

Transplanted cells, tissues or organs are protected from rejection by coating the transplanted tissue or organ with tethered Fas ligand. A fusion protein is made as described above which contains the soluble Fas ligand polypeptide fused to a cell-specific receptor ligand, e.g., insulin or integrin. In one embodiment, soluble Fas ligand is fused to insulin, and islet cells to be transplanted to a recipient subject are treated with the Fas ligand-insulin fusion protein. Insulin binds to its receptor on the islet cells, coating the islet cells with Fas ligand which provides a barrier to rejection. In another embodiment, an organ to be transplanted is perfused with a Fas ligand-integrin fusion protein such that the endothelium of the vasculature is coated with Fas ligand to stop reactive lymphocytes from entering the organ tissue.

Soluble Fas ligand may also be directly injected into a site of autoimmune inflammation, e.g., joint space or central nervous system, to attenuate autoimmune disease, e.g., rheumatoid arthritis or multiple sclerosis. Transfected autologous cells such as fibroblasts expressing the Fas ligand may also be administered into a site of autoimmune inflammation as a therapy for localized inflammatory autoimmune diseases.

The method of the invention can be used to induce tolerance, either to a foreign protein such as an allergen, or to a transplanted cell, tissue or organ. In one embodiment, autologous cells such as fibroblasts are co-transfected with the gene encoding the Fas ligand and a foreign protein, such as an allergen or autoantigen. The autologous cells would be administered to induce tolerance to the foreign protein. In another embodiment, transplant recipients are pre-tolerized to transplanted cells, tissues or organs by administration of allogeneic cells from the same source as the transplant tissue which express the Fas ligand. For example, transfected donor-derived fibroblasts expressing Fas ligand are injected into the recipient prior to transplanting the cells, tissues or organs from the same donor.

### Example 14. Gene Therapy

The present invention includes gene therapy with a gene encoding intact or soluble Fas ligand. The Fas ligand gene can also be used to provide protection of the virus vectors or foreign proteins used in gene therapies from destruction by the immune system of the recipient subject. Gene therapy is used to provide local immunosuppression by introduction of the Fas ligand polynucleotide into appropriate cells. The Fas ligand polynucleotide sequence may be linked to a tissue specific promoter ensuring Fas ligand expression only in the tissue for which protection is desired. Delivery of Fas ligand polynucleotide can be achieved using a recombinant expression vector such as a chimeric virus or a colloidal dispersion system. Especially preferred for therapeutic delivery of polynucleotide sequences is the use of targeted liposomes.

Various viral vectors which can be utilized for gene therapy as taught herein include adenovirus, herpes virus, vaccinia, or an RNA virus such as a retrovirus. When an RNA virus is used, preferably it is a derivative of a murine or avian retrovirus. Examples of retroviral vectors in which a single foreign gene can be inserted include, but are not limited to: Moloney murine leukemia virus (MoMuLV), Harvey murine sarcoma virus (HaMuSV), murine mammary tumor virus (MuMTV), and Rous Sarcoma Virus (RSV). Preferably, when the subject is human, a vector such as gibbon ape leukemia virus (GalLV) is utilized. A number of additional retroviral vectors can incorporate multiple genes. All of these vectors can transfer or incorporate a gene for a selectable marker so that transduced cells can be identified and generated. By inserting a Fas ligand polynucleotide into the viral vector, along with another gene which encodes a ligand for a receptor on a specific target cell, for example, a vector is now target specific. Retroviral vectors can be made target specific by attaching, for example, a sugar, a glycolipid, or a protein. Preferred targeting is accomplished by using an antibody to target the retroviral vector. Those of skill in the art will know of, or can readily ascertain without undue experimentation, specific polynucleotide sequences with can be inserted into the retroviral genome or attached to a viral envelope to allow target specific delivery of the retroviral vector containing the Fas ligand polynucleotide.

The Fas ligand gene can also be combined with another gene by inserting both a Fas ligand polynucleotide and a gene used for therapeutic purposes into the viral vector. Expression of the Fas ligand provides immunoprotection of the viral vector and/or foreign gene from destruction by the recipient host.

Since recombinant retroviruses are defective, they require assistance in order to produce infectious vector particles. This assistance can be provided, for example, by using helper cell lines that contain plasmids encoding all the structural genes of the retrovirus under the control of regulatory sequences within the LTR. These plasmids are missing a nucleotide sequence which enables the packaging mechanism to recognize an RNA transcript for encapsidation. Helper cell lines which have deletions of the packaging signal include, but are not limited to Ψ2, PA317 and PA12, for example. These cell lines produce empty virions, since no genome is packaged. If a retroviral vector is introduced into such cells in which the packaging signal is intact, but the structural genes are replaced by other genes of interest, the vector can be packaged and vector virion produced.

Alternatively, NIH 3T3 or other tissue culture cells can be directly transfected with plasmids encoding the retroviral structural genes *gag, pol* and *env*, by conventional calcium phosphate transfection. These cells are then transfected with the vector plasmid containing the Fas ligand gene. The resulting cells release the retroviral vector into the culture medium.

Another targeted delivery system for Fas ligand polynucleotide is a colloidal dispersion system. Colloidal dispersion systems include macromolecule complexes, nanocapsules, microspheres, beads, and lipid-based systems including oil-in-water emulsions, micelles, mixed micelles, and liposomes. The preferred colloidal system of the invention is a liposome. Liposomes are artificial membrane vesicles that are useful as delivery vehicles *in vitro* and *in vivo*. It has been shown that large unilamellar vehicles (LUV), which range in size from 0.2 - 4.0 µm can encapsulate a substantial percentage of an aqueous buffer containing large macromolecules. RNA, DNA and intact virions can be encapsulated within the aqueous interior and be delivered to cells in a biologically active form (Fraley et al. (1981) Trends Biochem. Sci. 6:77). In order for a liposome to be an efficient gene transfer vehicle, the following characteristics should be present: (1) encapsulation of the Fas ligand polynucleotide at high efficiency while not compromising biological activity; (2) preferential and substantial binding to a target cell in comparison to a non-target cell; (3) delivery of the aqueous contents of the vesicle to the target cell cytoplasm at high efficiency; and (4) accurate and effective expression of genetic information (Manninno et al. (1988) Biotechniques 6:682).

The composition of the liposome is usually a combination of phospholipid, particularly high-phase-transition-temperature phospholipids, usually in combination with sterols, especially cholesterol. Other phospholipids or other lipids may be used. The physical characteristics of liposomes depend on pH, ionic strength, and the presence of divalent cations.

The targeting of liposomes can be classified based on anatomical and mechanistic factors. Anatomical classification is based on the level of selectivity, for example, organ-specific, cell-specific, and organelle-specific. Mechanistic targeting can be distinguished based upon whether it is passive or active. Passive targeting utilizes the natural tendency of liposomes to distribute to cells of the reticulo-endothelial system (RES) in organs which contain sinusoidal capillaries. Active targeting, on the other hand, involves alteration of the liposome by coupling the liposome to a specific ligand such as a monoclonal antibody, sugar, glycolipid, or protein, or by changing the composition or size of the liposome in order to achieve targeting to organs and cell types other than the naturally occurring sites of localization.

The invention is shown herein in what is considered to be the most practical and preferred embodiments. It is recognized, however, that departures may be made therefrom which are within the scope of the invention and that obvious modifications will occur to one skilled in the art upon reading this disclosure.

**TABLE 1. ALLOGRAFT TRANSPLANTATION OF INTACT TESTIS TISSUE AND ISOLATED SERTOLI CELLS FROM C57B1/6 (B6), B6 MRL-lpr (B6-lpr) and B6Smn.C3H-gld (B6-gld) INTO Balb/c MICE.**

| DONOR | Fas Ligand | Graft | Accepted | Rejected | Rejection Rate |
|---|---|---|---|---|---|
| B6 | functional | Testis | 20 | 0 | 0 % |
| | | Sertoli | 7 | 0 | 0 % |
| B6-lpr | functional | Testis | 3 | 0 | 0 % |
| | | Sertoli | 3 | 0 | 0 % |
| B6-gld | non-functional | Testis | 0 | 25 | 100 % |
| | | Sertoli | 1 | 11 | 92 % |

**TABLE 2. EFFECT OF ALLOGRAFT REJECTION OF NON-FAS LIGAND BEARING ON FAS LIGAND BEARING TISSUE**

| DONOR | CHALLENGE | GRAFT | ACCEPTED | REJECTED | REJECTION RATE |
|---|---|---|---|---|---|
| B6 | NONE | Testis | 4 | 0 | 0 % |
| | | Sertoli | 2 | 0 | 0 % |
| B6 | B6 spleen cells | Testis | 4 | 0 | 0 % |
| | | Sertoli | 2 | 0 | 0 % |

## Claims

1. The use of a Fas ligand for the manufacture of a medicament for suppressing T-lymphocyte-mediated transplant rejection in a recipient mammal.

2. The use of a cell which expresses a Fas ligand for the manufacture of a medicament for suppressing T-lymphocyte-mediated graft rejection in a recipient mammal.

3. The use of a Fas ligand, or a cell which expresses a Fas ligand, for the manufacture of a medicament for suppressing or preventing T-lymphocyte-mediated disease recurrence in a mammal.

4. The use of a Fas ligand, or a cell which expresses the Fas ligand, for the manufacture of a medicament for treating a T-lymphocyte-mediated disease in a mammal.

5. The use according to any one of claims 2 to 4, wherein the cell has been transfected with and expresses a polynucleotide encoding a biologically active Fas ligand.

6. The use according to any one of claims 2 to 5, wherein said cell is a cell of a graft.

7. The use according to any one of claims 2 to 5, wherein the cell is not a part of a said graft.

8. The use according to any one of claims 2 to 5, wherein the cell is an islet cell.

9. The use of a polynucleotide encoding a Fas ligand for the manufacture of a medicament for suppressing T-lymphocyte-mediated graft rejection in a recipient mammal.

10. The use of a polynucleotide encoding a Fas ligand for the manufacture of a medicament for suppressing or preventing T-lymphocyte-mediated disease recurrence in a mammal.

11. The use of a polynucleotide encoding a Fas ligand for the manufacture of a medicament for treating a T-lymphocyte-mediated disease in a mammal.

12. The use according to any one of claims 1 to 11, wherein the mammal has a T-lymphocyte-mediated diabetic disease.

13. The use according to any one of claims 1 to 12, wherein the Fas ligand is a membrane-associated Fas ligand.

14. A method for improving transplantation acceptance, the method comprising:
determining expression of Fas ligand in a donor tissue; and
selecting donor tissue expressing Fas ligand for transplantation.

15. A method for improving transplantation acceptance, the method comprising:
determining expression of Fas ligand in a recipient tissue; and
selecting in a recipient subject a site for transplantation from recipient tissue
expressing Fas ligand.

16. A method of creating an immunologically-privileged site in a recipient mammal, the method comprising coating a cell, tissue or organ to be transplanted with a Fas ligand prior to transplantation.

## Patentansprüche

1. Verwendung eines Fas-Liganden zur Herstellung eines Arzneimittels zur Unterdrückung von T-Lymphozyten-vermittelter Transplantat-Abstoßung bei einem Empfänger-Säugetier.

2. Verwendung einer Zelle, die einen Fas-Liganden exprimiert, zur Herstellung eines Arzneimittels zur Unterdrückung von T-Lymphozyten-vermittelter Abstoßung von transplantiertem Gewebe bei einem Empfänger-Säugetier.

3. Verwendung eines Fas-Liganden oder einer Zelle, die einen Fas-Liganden exprimiert, zur Herstellung eines Arzneimittels zur Unterdrückung oder Verhütung eines T-Lymphozyten-vermittelten Wiederauftretens einer Krankheit bei einem Säugetier.

4. Verwendung eines Fas-Liganden oder einer Zelle, die den Fas-Liganden exprimiert, zur Herstellung eines Arzneimittels zur Behandlung einer T-Lymphozyten-vermittelten Krankheit bei einem Säugetier.

5. Verwendung nach einem der Ansprüche 2 bis 4, bei der die Zelle mit einem Polynukleotid, das einen biologisch aktiven Fas-Liganden kodiert, transfiziert wurde und ihn exprimiert.

6. Verwendung nach einem der Ansprüche 2 bis 5, bei der die Zelle eine Zelle eines transplantierten Gewebes ist.

7. Verwendung nach einem der Ansprüche 2 bis 5, bei der die Zelle nicht ein Teil des transplantierten Gewebes ist.

8. Verwendung nach einem der Ansprüche 2 bis 5, bei der die Zelle eine Inselzelle ist.

9. Verwendung eines Polynukleotids, das einen Fas-Liganden kodiert, zur Herstellung eines Arzneimittels zur Unterdrückung von T-Lymphozyten-vermittelter Abstoßung von transplantiertem Gewebe bei einem Empfänger-Säugetier.

10. Verwendung eines Polynukleotids, das einen Fas-Liganden kodiert, zur Herstellung eines Arzneimittels zur Unterdrückung oder Verhütung eines T-Lymphozyten-vermittelten Wiederauftretens einer Krankheit bei einem Säugetier.

11. Verwendung eines Polynukleotids, das einen Fas-Liganden kodiert, zur Herstellung eines Arzneimittels zur Behandlung einer T-Lymphozyten-vermittelten Krankheit bei einem Säugetier.

12. Verwendung nach einem der Ansprüche 1 bis 11, bei der das Säugetier eine T-Lymphozyten-vermittelte diabetische Krankheit hat.

13. Verwendung nach einem der Ansprüche 1 bis 12, bei der der Fas-Ligand ein Membran-verbundener Fas-Ligand ist.

14. Verfahren zur Verbesserung der Transplantations-Akzeptanz, wobei das Verfahren aufweist:
Bestimmen der Expression von Fas-Ligand in einem Donorgewebe: und
Auswählen von Fas-Ligand exprimierendem Donorgewebe zur Transplantation.

15. Verfahren zur Verbesserung der Transplantations-Akzeptanz, wobei das Verfahren aufweist:
Bestimmen der Expression von Fas-Ligand in einem Empfängergewebe; und
Auswählen bei einem Empfänger-Individuum einer Stelle zur Transplantation aus Fas-Ligand exprimierendem Empfängergewebe.

16. Verfahren zur Schaffung einer immunologisch privilegierten Stelle bei einem Empfänger-Säugetier, wobei das Verfahren das Beschichten einer Stelle, eines Gewebes oder eines Organs, die (das) transplantiert werden soll, mit einem Fas-Liganden vor der Transplantation aufweist.

## Revendications

1. Emploi d'un ligand Fas en vue de la fabrication d'un médicament conçu pour supprimer, chez un mammifère receveur, le rejet de greffe médié par les lymphocytes T.

2. Emploi d'une cellule exprimant un ligand Fas en vue de la fabrication d'un médicament conçu pour supprimer, chez un mammifère receveur, le rejet de greffon médié par les lymphocytes T.

3. Emploi d'un ligand Fas, ou d'une cellule exprimant un ligand Fas, en vue de la fabrication d'un médicament conçu pour supprimer ou prévenir, chez un mammifère, une rechute de maladie médiée par les lymphocytes T.

4. Emploi d'un ligand Fas, ou d'une cellule exprimant un ligand Fas, en vue de la fabrication d'un médicament conçu pour le traitement, chez un mammifère, d'une maladie médiée par les lymphocytes T.

5. Emploi conforme à l'une des revendications 2 à 4, pour lequel la cellule a été transfectée avec un polynucléotide codant un ligand Fas bioactif et dans lequel elle exprime ce polynucléotide.

6. Emploi conforme à l'une des revendications 2 à 5, dans lequel ladite cellule est une cellule de greffon.

7. Emploi conforme à l'une des revendications 2 à 5, dans lequel ladite cellule ne fait pas partie d'un greffon.

8. Emploi conforme à l'une des revendications 2 à 5, dans lequel ladite cellule est une cellule d'îlots de Langerhans.

9. Emploi d'un polynucléotide codant un ligand Fas en vue de la fabrication d'un médicament conçu pour supprimer, chez un mammifère receveur, le rej et de greffe médié par les lymphocytes T.

10. Emploi d'un polynucléotide codant un ligand Fas en vue de la fabrication d'un médicament conçu pour supprimer ou prévenir, chez un mammifère, une rechute de maladie médiée par les lymphocytes T.

11. Emploi d'un polynucléotide codant un ligand Fas en vue de la fabrication d'un médicament conçu pour le traitement, chez un mammifère, d'une maladie médiée par les lymphocytes T.

12. Emploi conforme à l'une des revendications 1 à 11, le mammifère étant atteint d'une maladie diabétique médiée par les lymphocytes T.

13. Emploi conforme à l'une des revendications 1 à 12, dans lequel le ligand Fas est un ligand Fas associé à la membrane.

14. Procédé ayant pour but d'améliorer la tolérance lors d'une transplantation, lequel procédé comporte :
- le fait de déterminer l'expression de ligand Fas dans un tissu de donneur,
- et le fait de sélectionner, pour la transplantation, un tissu de donneur exprimant un ligand Fas.

15. Procédé ayant pour but d'améliorer la tolérance lors d'une transplantation, lequel procédé comporte :
- le fait de déterminer l'expression de ligand Fas dans un tissu de receveur,
- et le fait de sélectionner, chez un receveur, pour la transplantation, un site dans un tissu de receveur exprimant un ligand Fas.

16. Procédé de création d'un site immunologiquement privilégié chez un mammifère receveur, lequel procédé comporte le fait de doter une cellule, un tissu ou un organe à transplanter, avant transplantation, d'un enrobage de ligand Fas.
